# EUROPEAN PATENT SPECIFICATION

(11) **EP 4 345 799 B1**
(45) Date of publication and mention of the grant of the patent: **25.12.2024**
(21) Application number: 23200038.0
(22) Date of filing: 27.09.2023
(51) Int. Cl.: G09B 23/28

(54) **A TOURNIQUET APPARATUS**
STAUBINDEVORRICHTUNG
APPAREIL DE GARROT

(30) Priority: 29.09.2022 US 202263411349 P
(43) Date of publication of application: 03.04.2024
(73) Proprietor: Simetri, Inc., Winter Park Florida 32792 (US)
(72) Inventor: Ellis, Trevor, Winter Park, 32792 (US)
(74) Representative: Brothers, Christopher Michael

(56) References cited:
- WO-A2-2021/041006
- US-A1- 2009 005 804
- US-A1- 2022 047 273

## Description

### BACKGROUND OF THE INVENTION

The present invention is directed to a tourniquet for controlling blood flow to a wound and more particularly to a training tourniquet for use in training tourniquet technique which prevents exertion of too much pressure to the limb of a subject about which the tourniquet is placed.

Medical personnel, whether nurses in a hospital, emergency medical technicians, medics in the field, and even police and fire personnel need training in attending to wounds. One technique that requires training is the staunching of bleeding. One common technique for staunching bleeding is the use of a tourniquet about a limb above the wound.

The proper use of a tourniquet requires training including the repetitive performance of the technique. While it is known to practice on a mannequin, it is best to practice tourniquet techniques on an actual person. However, until the practitioner perfects their technique, such practice often results in over tightening of the tourniquet. This causes pain and even bruising to the person upon whom the technique is being performed. (WO 2021/041006).

Accordingly, a system which overcomes the shortcomings of the prior art is desired.

### SUMMARY OF THE INVENTION

The present invention relates to a tourniquet apparatus as defined in independent claim 1 and to a method of using the tourniquet apparatus as defined in independent method claim 11.

According to a first aspect of the present invention, there is provided a tourniquet apparatus comprising a platform; a first strap connected to the platform, the first strap being adapted to locate the platform on an appendage of a person; a first support; a second support, at least one of the first and second supports being slidably disposed on the platform; a force-absorbing device having a first end and a second end, the first end being connected to the first support and the second end being connected to the second support; a second strap having opposite ends respectively connected to the first support and to the second support; a force-applying device operatively coupled to the second strap; whereby applying a force by way of the force-applying device causes a distance between the first support and the second support to increase; and a sensor disposed between the second strap and the platform measuring a compression force applied by the second strap to the sensor.

According to a second aspect of the present invention, there is provided a method of using a tourniquet apparatus according to the first aspect above, the method comprising applying force by way of the force-applying device and thereby causing a distance between the first support and second support to increase, the force-absorbing device absorbing the applied force, and measuring with a sensor the force applied by the second strap to the sensor.

Preferably, the torniquet device is a pressure limited training torniquet to train medical personnel and the like to use a tourniquet correctly.

Preferably, both the first and second supports are slidably disposed on the platform.

Preferably, the force absorbing device is in the form of one or more resilient devices such as one or more springs.

Preferably, the force-applying device is a windlass.

Advantageously, a sensor cover, to protect the sensor, is disposed between the sensor and the second strap. The pressure applied to the sensor cover is applied in turn to the sensor.

### BRIEF DESCRIPTION OF THE DRAWINGS

In order that the present invention can be clearly and completely disclosed, reference will now be made, by way of example only, to the accompanying drawings, in which:-
Figure 1 is a top perspective view of a tourniquet apparatus;
Figure 1A is a top perspective view of a sensor disposed on the tourniquet apparatus;
Figure 2 is a bottom perspective view of the tourniquet apparatus; and
Figure 2A is a bottom perspective view of the tourniquet apparatus.

### DETAILED DESCRIPTION OF THE PREFERRED EMBODIMENTS

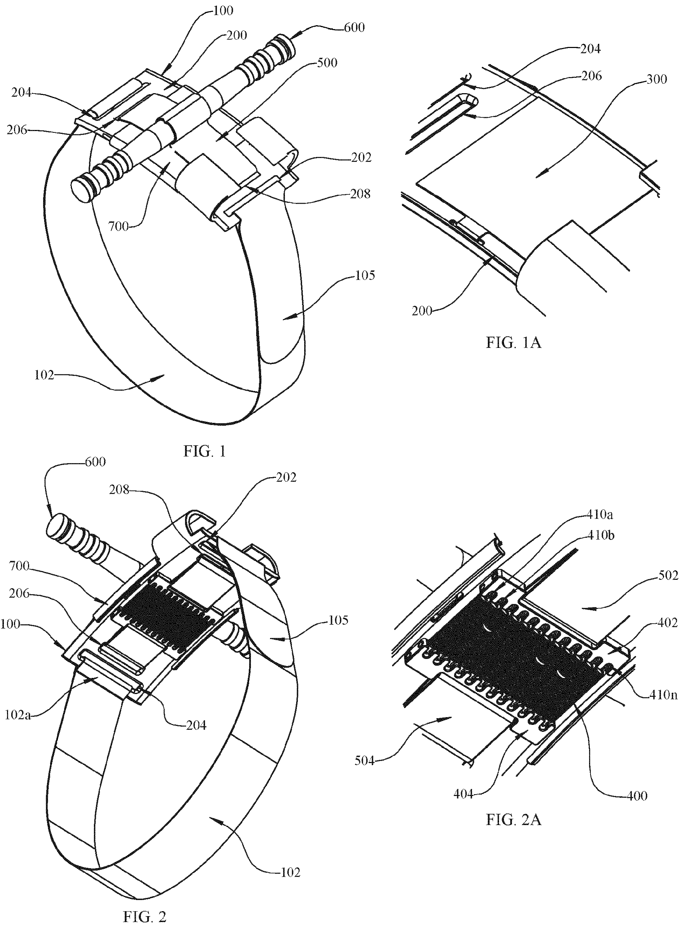

Reference is now made to Figures 1 and 2 in which a tourniquet apparatus in the form of a pressure limited training tourniquet, generally indicated as 100 is shown. Tourniquet 100 includes a platform 200 having receiving slots 202, 204 for receiving ends 102a, 105 of a first strap 102 therethrough. In this way, platform 200 and the first strap 102 encircle a limb of a person being treated or a training patient. As known in the art, end 102a is fixedly secured to the platform 200 at the receiving slot 204 and end 105 secures upon itself using Velcro° fastener or some other fastening means at the receiving slot 202 to enable adjusting of the length of the first strap 102 to snugly fit each person being treated.

Referring to Figure 2A, a first support 402 is slidably disposed on a limb facing surface of the platform 200. A second support 404 is also slidably disposed on a limb facing surface of the platform 200 spaced from first support 402. A force absorbing device 410 in the form of resilient means such as at least one spring, and preferably a plurality of springs 410a-410n, are supported at a first end thereof by the first support 402 and at a second end thereof by the second support 404. In this way, springs 410a-410n apply a force biasing the first and second supports 402, 404 towards each other and when either or both of the first and second supports 402, 404 are moved away from each other, springs 410a-410n are stretched, and they become elongated.

The platform 200 is formed with a third receiving slot 206 therein and a fourth receiving slot 208 therein. A second strap 500 extends through third receiving slot 206 and the fourth receiving slot 208. The second strap 500 is connected to the first support 402 at a first end 502 of the second strap 500 and connected to the second support 404 at a second end 504 of the second strap 500. A force-applying device preferably in the form of a windlass 600 (as shown) is operatively coupled to the second strap 500 to tighten the second strap 500 relative to platform 200 and apply a compression force.

A sensor 300, preferably a pressure sensor, for measuring the force applied thereto is disposed on a surface of the platform 200 facing away from the limb (see Figure 1A) between the platform 200 and a sensor cover 700. As the windlass 600 tightens the second strap 500, the second strap 500 exerts a force in the direction of the sensor 300 measured as a pressure about the limb. Sensor cover 700 is disposed between the second strap 500 and sensor 300 to protect sensor 300, but is sufficiently pliable to transmit the force applied to cover 700 to sensor 300.

At the same time, as the windlass 600 tightens strap 500, strap 500 exerts a force at the ends 502, 504 of the second strap 500. The second strap 500 is in effect pulled towards the sensor 300. Once the force exceeds a predetermined amount, determined by the strength of the springs 410a-410n, the ends 502, 504 coupled to respective first and second supports 402, 404 pull the first and second supports 402, 404 apart increasing the distance between them, and stretching the springs 410a-410n. The sensor 300 experiences and measures the applied force, but the limb of the test subject does not. The force is "transferred" from the first strap 102, where it would normally be expressed to the limb of the person being treated, to the springs 410 which absorb the force in a way that it is not felt by the person being treated, but capable of enabling the trainee to obtain feedback regarding their tightening techniques. In this way, the tightening is not felt by the person being treated, so that no bruising or pain occurs.

The sensor 300 is connected to a display, either visual and/or audible( not shown), which provides feedback to the user when the trainee has applied the appropriate number of turns to the windlass 600. In other words, the user is notified when pain would have normally been experienced by the person to whom the torniquet is applied, or similarly, when sufficient pressure would have been applied to staunch bleeding.

## Claims

1. A tourniquet apparatus (100) comprising:
a platform (200);
a first strap (102) connected to the platform (200), the first strap (102) being adapted to locate the platform (200) on an appendage of a person;
a first support (402); a second support (404), at least one of the first and second supports being slidably disposed on the platform (200);
a force-absorbing device (410) having a first end and a second end; the first end being affixed to the first support (402) and the second end being affixed to the second support (404);
a second strap (500) having opposite ends respectively connected to the first support (402) and to the second support (404);
a force-applying device (600) operatively coupled to the second strap (500); whereby applying a force by way of the force-applying device (600) causes a distance between the first support (402) and the second support (404) to increase; and
a sensor (300) disposed between the second strap (500) and the platform (200) measuring a compression force applied by the second strap (500) to the sensor (300).

2. The tourniquet apparatus (100) according to claim 1, and further comprising a cover (700) disposed between the second strap (500) and the sensor (300), the cover (700) transmitting a force from the second strap (500) to the sensor (300).

3. The tourniquet apparatus (100)according to claim 1 or 2, wherein the platform (200) further comprises:
at least one receiving slot (202) for receiving at least a first end (105) of at least the first strap (102).

4. The tourniquet apparatus (100) of any preceding claim, and further comprising a fastening means for connecting the first strap (102) to the platform (200).

5. The tourniquet apparatus (100) of any preceding claim, wherein the sensor (300) is disposed facing away from the appendage of the person upon which the tourniquet apparatus (100) is disposed.

6. The tourniquet apparatus (100) of any preceding claim, wherein the first strap (102) encircles the appendage of the person.7.

7. The tourniquet apparatus (100) of any preceding claim, wherein at least the first support (402) is disposed on an appendage-facing surface of the platform (200).

8. The tourniquet apparatus (100) of any preceding claim, wherein the sensor (300) operatively communicates with a display, the display providing feedback when the sensor (300) recognizes an appropriate amount of force has been applied by the force-applying device (600).

9. The tourniquet apparatus (100) of claim 8, wherein the display is visual.

10. The tourniquet apparatus (100) of claim 8 or 9, wherein the display is audible.

11. A method of using a tourniquet apparatus (100) according to any one of claims 1 to 10, the method comprising applying force by way of the force-applying device (600) and thereby causing a distance between the first support (402) and second support (404) to increase, the force-absorbing device absorbing the applied force, and measuring with a sensor (300) the force applied by the second strap (500) to the sensor (300).

12. A method according to claim 11, wherein the applied force is transferred from the first strap (102) to the force absorbing device (410) whereby the applied force is not felt by a person wearing the tourniquet apparatus (100).

13. A method according to claim 12, and further comprising the user being given training in use of the tourniquet apparatus (100) obtaining visual or audio feedback from a display.

## Patentansprüche

1. Eine Tourniquet-Vorrichtung (100) umfassend:
Eine Plattform (200);
einen ersten Riemen (102), der mit der Plattform (200) verbunden ist, der erste Riemen (102) ist so angepasst, dass er die Plattform (200) an einer Gliedmaße einer Person positionieren kann;
eine erste Halterung (402); eine zweite Halterung (404), wobei mindestens entweder die erste oder die zweite Halterung verschiebbar auf der Plattform (200) angeordnet ist;
eine kraftaufnehmende Vorrichtung (410) mit einem ersten Ende und einem zweiten Ende; das erste Ende ist an der ersten Halterung (402) und das zweite Ende an der zweiten Halterung (404) befestigt;
einen zweiten Riemen (500) mit gegenüberliegenden Enden, die mit der ersten Halterung (402) beziehungsweise der zweiten Halterung (404) verbunden sind;
eine kraftanwendende Vorrichtung (600), die wirkend mit dem zweiten Riemen (500) gekoppelt ist; wodurch das Anwenden einer Kraft über die kraftanwendende Vorrichtung (600) einen Abstand zwischen der ersten Halterung (402) und der zweiten Halterung (404) vergrößert; und
einen Sensor (300), der zwischen dem zweiten Riemen (500) und der Plattform (200) angeordnet ist und eine Kompressionskraft misst, die durch den zweiten Riemen (500) auf den Sensor (300) angewendet wird.

2. Tourniquet-Vorrichtung (100) nach Anspruch 1, und ferner umfassend eine Abdeckung (700), die zwischen dem zweiten Riemen (500) und dem Sensor (300) angeordnet ist, wobei die Abdeckung (700) eine Kraft von dem zweiten Riemen (500) auf den Sensor (300) überträgt.

3. Tourniquet-Vorrichtung (100) nach Anspruch 1 oder 2, wobei die Plattform (200) ferner umfasst:
Mindestens eine Aufnahmenut (202), um mindestens ein erstes Ende (105) mindestens des ersten Riemens (102) aufzunehmen.

4. Tourniquet-Vorrichtung (100) nach einem der vorstehenden Ansprüche und ferner umfassend ein Befestigungsmittel für das Verbinden des ersten Riemens (102) mit der Plattform (200).

5. Tourniquet-Vorrichtung (100) nach einem der vorstehenden Ansprüche, wobei der Sensor (300) so angeordnet ist, dass er von der Gliedmaße der Person, an der die Tourniquet-Vorrichtung (100) angewendet wird, weg zeigt.

6. Tourniquet-Vorrichtung (100) nach einem der vorstehenden Ansprüche, wobei der erste Riemen (102) die Gliedmaße der Person umfasst.

7. Tourniquet-Vorrichtung (100) nach einem der vorstehenden Ansprüche, wobei mindestens die erste Halterung (402) an einer zu der Gliedmaße zeigenden Fläche der Plattform (200) angeordnet ist.

8. Tourniquet-Vorrichtung (100) nach einem der vorstehenden Ansprüche, wobei der Sensor (300) wirkend mit einer Anzeige kommuniziert, wobei die Anzeige ein Feedback liefert, wenn der Sensor (300) erkennt, dass ein entsprechender Kraftbetrag über die kraftanwendende Vorrichtung (600) angewendet wurde.

9. Tourniquet-Vorrichtung (100) nach Anspruch 8, wobei die Anzeige visuell ist.

10. Tourniquet-Vorrichtung (100) nach Anspruch 8 oder 9, wobei die Anzeige akustisch ist.

11. Verfahren für das Verwenden einer Tourniquet-Vorrichtung (100) nach einem der Ansprüche 1 bis 10, das Verfahren umfassend das Anwenden einer Kraft über die kraftanwendende Vorrichtung (600) und dadurch das Veranlassen, dass ein Abstand zwischen der ersten Halterung (402) und der zweiten Halterung (404) vergrößert wird, wobei die kraftaufnehmende Vorrichtung die angewendete Kraft aufnimmt und mit einem Sensor (300) die Kraft misst, die von dem zweiten Riemen (500) auf den Sensor (300) angewendet wird.

12. Verfahren nach Anspruch 11, wobei die angewendete Kraft von dem ersten Riemen (102) auf die kraftaufnehmende Vorrichtung (410) übertragen wird, wodurch die angewendete Kraft von einer Person, die die Tourniquet-Vorrichtung (100) trägt, nicht gespürt wird.

13. Verfahren nach Anspruch 12 und ferner umfassend eine Schulung des Benutzers in der Verwendung der Tourniquet-Vorrichtung (100), bei der er visuelles oder akustisches Feedback von einer Anzeige erhält.

## Revendications

1. Appareil de garrot (100) comprenant :
une plateforme (200) ;
une première sangle (102) reliée à la plateforme (200), la première sangle (102) étant adaptée à placer la plateforme (200) sur un appendice d'une personne ;
un premier support (402) ; un second support (404), au moins l'un des premier et second supports étant disposé de manière coulissante sur la plateforme (200) ;
un dispositif d'absorption de force (410) présentant une première extrémité et une seconde extrémité, la première extrémité étant fixée au premier support (402) et la seconde extrémité étant fixée au second support (404) ;
une seconde sangle (500) présentant des extrémités opposées reliées respectivement au premier support (402) et au second support (404) ;
un dispositif d'application de force (600) accouplé de manière fonctionnelle à la seconde sangle (500) ; grâce à quoi l'application d'une force au moyen du dispositif d'application de force (600) provoque une augmentation de la distance entre le premier support (402) et le second support (404) ; et
un capteur (300) disposé entre la seconde sangle (500) et la plateforme (200) mesure une force de compression appliquée par la seconde sangle (500) au capteur (300).

2. Appareil de garrot (100) selon la revendication 1, et comprenant en outre un couvercle (700) disposé entre la seconde sangle (500) et le capteur (300), le couvercle (700) transmettant une force de la seconde sangle (500) au capteur (300).

3. Appareil de garrot (100) selon la revendication 1 à 2, dans lequel la plateforme (200) comprend en outre :
au moins une fente de réception (202) destinée à recevoir au moins une première extrémité (105) d'au moins la première sangle (102).

4. Appareil de garrot (100) selon une quelconque revendication précédente, et comprenant en outre un moyen de fixation destiné à relier la première sangle (102) à la plateforme (200).

5. Appareil de garrot (100) selon une quelconque revendication précédente, dans lequel le capteur (300) est disposé orienté à l'opposé de l'appendice de la personne sur laquelle l'appareil de garrot (100) est disposé.

6. Appareil de garrot (100) selon une quelconque revendication précédente, dans lequel la première sangle (102) encercle l'appendice de la personne.

7. Appareil de garrot (100) selon une quelconque revendication précédente, dans lequel au moins le premier support (402) est disposé sur une surface faisant face à un appendice de la plateforme (200).

8. Appareil de garrot (100) selon une quelconque revendication précédente, dans lequel le capteur (300) communique de manière fonctionnelle avec un affichage, l'affichage fournissant une rétroaction lorsque le capteur (300) reconnaît qu'une quantité appropriée de force a été appliquée par le dispositif d'application de force (600).

9. Appareil de garrot (100) selon la revendication 8, dans lequel l'affichage est visuel.

10. Appareil de garrot (100) selon la revendication 8 ou 9, dans lequel l'affichage est audible.

11. Procédé d'utilisation d'un appareil de garrot (100) selon l'une quelconque des revendications 1 à 10, le procédé comprenant l'application d'une force au moyen du dispositif d'application de force (600) et provoquant ainsi une augmentation de la distance entre le premier support (402) et le second support (404), le dispositif d'absorption de force absorbant la force appliquée et la mesure avec un capteur (300) de la force appliquée par la seconde sangle (500) au capteur (300).

12. Procédé selon la revendication 11, dans lequel la force appliquée est transférée de la première sangle (102) au dispositif d'absorption de force (410), grâce à quoi la force appliquée n'est pas ressentie par une personne portant l'appareil de garrot (100).

13. Procédé selon la revendication 12, et comprenant en outre l'apprentissage de l'utilisateur lors de l'utilisation de l'appareil de garrot (100) en obtenant une rétroaction visuelle ou audio à partir d'un affichage.
